(19) **Europäisches Patentamt**

**European Patent Office**

**Office européen des brevets**

(11) **EP 1 039 930 B1**

(12) **EUROPEAN PATENT SPECIFICATION**

(45) Date of publication and mention
of the grant of the patent:
**23.11.2005 Bulletin 2005/47**

(51) Int Cl.⁷: **A61K 39/39**

(86) International application number:
**PCT/EP1998/008562**

(21) Application number: **98966705.0**

(22) Date of filing: **18.12.1998**

(87) International publication number:
**WO 1999/033488 (08.07.1999 Gazette 1999/27)**

(54) **ADJUVANTED VACCINE COMPRISING AN ANTIGEN FROM STREPTOCOCCUS PNEUMONIAE AND A CpG OLIGONUCLEOTIDE**

ADJUVIERTE IMPFSTOFF ENTHALTEND STREPTOCOCCUS PNEUMONIAE ANTIGEN UND CPG OLIGONUKLEOTID

VACCIN POUR STREPTOCOCCUS PNEUMONIAE COMPRENANT COMME ADJUVANT UN OLIGONUCLEOTIDE CpG

(84) Designated Contracting States:
**AT BE CH CY DE DK ES FI FR GB GR IE IT LI LU MC NL PT SE**
Designated Extension States:
**SI**

(30) Priority: **24.12.1997 GB 9727262**

(43) Date of publication of application:
**04.10.2000 Bulletin 2000/40**

(73) Proprietor: **Glaxosmithkline Biologicals S.A.
1330 Rixensart (BE)**

(72) Inventors:
- **DALEMANS, Wilfried, L., J.**
  **B-1330 Rixensartxensart (BE)**
- **LAFERRIERE, Craig, A. J.**
  **B-1330 Rixensart (BE)**
- **PRIEELS, Jean-Paul**
  **B-1330 Rixensart (BE)**

(74) Representative:
**Dalton, Marcus Jonathan William et al
GlaxoSmithKline
Corporate Intellectual Property (CN9.25.1)
980 Great West Road
Brentford, Middlesex TW8 9GS (GB)**

(56) References cited:
**EP-A- 0 497 525          EP-A- 0 855 184
WO-A-98/37919**

- **R.S. CHU ET AL.: "CpG OLIGODEOXYNUCLEOTIDES ACT AS ADJUVANTS THAT SWITCH ON T HELPER 1 (Th1) IMMUNITY." JOURNAL OF EXPERIMENTAL MEDICINE, vol. 186, no. 10, 17 November 1997, pages 1623-1631, XP002910130 NEW YORK, N.Y., US**
- **A.M. KRIEG ET AL.: "CpG MOTIFS IN BACTERIAL DNA TRIGGER DIRECT B-CELL ACTIVATION." NATURE, vol. 374, 6 April 1995, pages 546-549, XP002106695 LONDON, GB cited in the application**
- **D.S. THREADGILL ET AL.: "MITOGENIC SYNTHETIC POLYNUCLEOTIDES SUPPRESS THE ANTIBODY RESPONSE TO A BACTERIAL POLYSACCHARIDE." VACCINE, vol. 16, no. 1, January 1998, pages 76-82, XP002911830 GUILDFORD, GB cited in the application**

**Description**

The present invention relates to new vaccine formulations, and to methods for their production and their use in medicine.

Immunomodulatory oligonucleotides containing unmethylated CpG dinucleotides ("CpG") are known (WO 96/02555, EP 468520). CpG is an abbreviation for cytosine-guanosine dinucleotide motifs present in DNA. Historically, it was observed that the DNA fraction of BCG could exert an anti-tumor effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity (Tokunaga, T. et al. Microbial. Immunol. 36: 55 (1992)). The central role of the CG motif in immunostimulation was later elucidated in a publication by Krieg (Nature 374 p546 1995). Chu et al. (J. Exp. Med., 1997,186:1623-1631) disclosed that CpG oligonucleotides can act as Th1 adjuvants. EP855184 discloses further CpG sequences for use in pharmaceutical compositions. WO 98/37919 discloses the use of CpG sequences in the treatment of LPS-associated disorders. Detailed analysis has shown that the CG motif has sequences that are common in bacterial DNA but are rare in vertebrate DNA.

It is currently believed that this evolutionary difference allows the vertebrate immune system to detect the presence of bacterial DNA (as occurring during an infection) leading consequently to the stimulation of the immune system. Immunostimulatory activity has been shown for sequences as small as 15 nucleotide bases (Krieg, et al. Nature 374: 546 (1995)) and that the CpG motif has to be unmethylated. It has been postulated that the oligo should be in a hexamer setting: purine purine CG pyrimidine pyrimidine, but this is not obligatory.

Streptococcus pneumoniae is a gram positive bacteria that is pathogenic for humans, causing invasive diseases such as pneumonia, bacteremia and meningitis, and diseases associated with colonisation, such as acute Otitis media. The mechanisms by which pneumococci spread to the lung, the cerebrospinal fluid and the blood is poorly understood. Growth of bacteria reaching normal lung alveoli is inhibited by their relative dryness and by the phagocytic activity of alveolar macrophages. Any anatomic or physiologic derangement of these co-ordinated defenses tends to augment the susceptibility of the lungs to infection. The cell-wall of Streptococcus pneumoniae has an important role in generating an inflammatory response in the alveoli of the lung (Gillespie et al , I&I 65: 3936). The release of cell-wall components occurs at the end of the pneumococcal growth cycle by autolysis due to the synthesis of the protein N-acetyl muramoyl-L-alanine amidase (lytA). DNA will also be released into the infected region upon autolysis of the pneumococci.

In order for the organism to have an effective immune response against invading bacteria, it must have mechanisms to coordinate the type of immune response most likely to stop infection. For intracellular pathogens, the coordination appears to occur between cell mediated or humoral immune responses, and these are controlled by T-cells of the type Th1 and Th2. However, extracellular bacteria frequently employ a polysaccharide either in the form of a capsule or a lipopolysaccharide to protect themselves from the effects of serum complement which can lyse the bacteria, or render them accessible to phagocytes such as macrophage and neutrophils.

In this case, the immune response follows another path, the T-independent immune response. The T-independent immune response may be further divided into Type 1 and Type 2. T-independent type 2 antigens possess the characteristics embodied by polysaccharide antigens, including: large molecular weight, repeat antigenic epitopes, ability to activate the complement cascade, poor in vivo degradability and inability to stimulate MHC class II dependent T cell help (Mond et al. Annu Rev Immunol 13:655-92). The Type 1 antigens, unlike the polysaccharides, are mitogenic for B-cells, and are comprised of the lipopolysaccharides (LPS). T-independent Type 2 antigens can not stimulate responses in neonatal mice or CBA/N mice that carry an X-linked immune B-cell defect (xid mice), whereas Type 1 antigens can.

Type 2 antigens induce weaker antibody responses as compared to T-dependent antigens such as proteins. Proteins are able to activate B-cells and induce the secretion of antibody by being processed into peptides and presented on the surface of the B-cell in the context of MHC class II, enabling the B-cell to interact with T-cells and receive additional signals required for maximal B-cell proliferation and maturation. However, whereas oligosaccharides may in some cases associate with MHC class II (Ishioka et al. J. Immunol. 148: 2446-2451) and lipidated polysaccharides appear to associate with CD1 present on lymphocytes, (Fairhurst, R.M. et al. Immunology Today 19: 257 (1998)), there is no known mechanism of presentation for Type 2 antigens to T cells.

Nevertheless, the multiple repeat nature of the polysaccharide polymer antigen can cause cross-linking of receptors on the B-cell surface, leading to B-cell activation by a mechanism not requiring T-cells. Thus polysaccharides are T-independent antigens and they are characterised in animals and human infants by the production of IgM antibodies, and the lack of boosting and immunological memory. It is only adult humans that can produce significant amounts of IgG antibody to most (but not all) polysaccharide antigens. The ability to switch antibody isotype to IgG coincides with the appearance of the complement receptor 2 (CR2) on the B-cells of infants or toddlers between 1.5 to 2 years of age, and this may provide the additional signal required for activation and maturation of B-cells.

The present invention in one aspect provides a vaccine formulation comprising a CpG oligonucleotide sequence and a polysaccharide antigen from Streptococcus pneumoniae which is capable of raising an immune response to a pneumococcal T independent antigen:

Production of IgG antibodies to the capsular polysaccharides of bacteria is essential because the principal mechanism of protection against these bacteria, complement mediated lysis and opsonophagocytosis, are most effective with this antibody isotype (Maslanka et al. Clin Diag Lab Immunol 4: 156-67, and Romero-Steiner et al. Clin Diag Lab Immunol 4: 415-22).

Polysaccharide antigen based vaccines are well known in the art, and four that have been licensed for human use include the Vi polysaccharide of *Salmonella typhi,* the PRP polysaccharide from *Haemophilus influenzae,* the tetravalent meningococcal vaccine composed of serotypes A, C, W 135 and Y, and the 23-Valent pneumococcal vaccine composed of the polysaccharides corresponding to serotypes 1, 2, 3,4, 5, 6B, 7F, 8, 9N, 9V, 10A, 11A, 12F, 14, 15B, 17F, 18C, 19A, 19F, 20, 22F, 23F, and 33.

The latter three vaccines confer protection against bacteria causing respiratory infections resulting in severe morbidity and mortality in infants, yet these vaccines have not been licensed for use in children less than two years of age because they are poorly immunogenic in this age group. EP497525 discloses a pneumococcal polysaccharide conjugate vaccine adjuvanted with alum.

The licensed polysaccharide vaccines listed above have different demonstrated clinical efficacy. The Vi polysaccharide vaccine has been estimated to have an efficacy between 55% and 77% in preventing culture confirmed typhoid fever (Plotkin and Cam, Arch Intern Med 155: 2293-99). The meningococcal C polysaccharide vaccine was shown to have an efficacy of 79% under epidemic conditions (De Wals P, et al. Bull World Health Organ. 74: 407-411). The 23-valent pneumococcal vaccine has shown a wide variation in clinical efficacy, from 0% to 81 % (Fedson et al. Arch Intern Med. 154: 2531-2535). The efficacy appears to be related to the risk group that is being immunised, such as the elderly, Hodgkin's disease, splenectomy, sickle cell disease and agammaglobulinemics (Fine et al Arch Intern Med. 154:2666-2677), and also to the disease manifestation. Pneumococcal pneumonia and Otitis media are diseases which do not have demonstrated protection by the 23-valent vaccine. It is generally accepted that the protective efficacy of the pneumococcal vaccine is more or less related to the concentration of antibody induced upon vaccination; indeed, the 23 polysaccharides were accepted for licensure solely upon the immunogenicity of each component polysaccharide (Ed. Williams et al. New York Academy of Sciences 1995 pp 241-249).

To increase the antibody response to the pneumococcal polysaccharides comprising the 23-valent vaccine, the present inventors tried to improve the immune response by addition of the immunostimulant QS21 EP 362 279 and dQS21 WO 96/33739; however, no increase in antibody responses to the polysaccharides in Rhesus monkeys could be measured.

Threadgill *et al* Vaccine 1998 Vol 16(1) p76 have recently reported that Immunostimulatory CpG oligonucleotides depress the polysaccharide specific antibody response when the oligonucleotide is formulated with Pseudomonas aeruginosa polysaccharide.

Surprisingly, the present inventors have found that it is possible to adjuvant the immune response to pneumococcal polysaccharide vaccines by formulating with an immunostimulatory CpG oligonucleotide, such formulations provided an immune response which produces significant levels of IgG antibodies.

According to the present invention there is provided a vaccine composition comprising a pneumococcal polysaccharide antigen adjuvanted by an Immunostimulatory CpG oligonucleotide.

The polysaccharide antigen may be unconjugated or conjugated to a carrier protein such that it provides T-helper epitopes.

The oligonucleotides may be DNA or RNA, but preferably contain a hexamer motif: purine purine CpG pyrimidine pyrimidine. More preferably the internucleotide linkage are modified to increase stability of the oligonucleotide. Preferred modifications are phosphorothioate linkages. The lytA protein involved in the catalytic degradation of the cell wall of pnemococci is produced at the time of autolysis, and is part of the competance induced operon (Mol. Microbiol 29:1125 (1998)). By definition the mRNA encoding lytA will be present in large quantities during synthesis of the lytA protein. Furthermore, the lytA protein contains a phosphoryl choline binding region that contains repeat DNA sequences (Yother and Briles J Bacteriol. 174: 601 (1992)), and which may be found on many other choline binding proteins present in Streptococci. The following CpG sequences were identified from the phosphoryl choline binding regions of lytA and from choline binding protein A (cbpA) (Rosenow et al. Mol. Microbiol 25: 819-829 (1997)).

## OLIGO 1: GCTACTGGT<u>ACG</u> <u>T</u>ACATTC AG<u>ACGG</u>C TCTT (lytA)

OLIGO 2: ACTATCTAA<u>ACGC</u>TAATGGTGCTATG<u>GCGA</u>CAGGATGGCT

·(cbpA)·

and may be utilised in the present invention.

The following oligonucleotide immunostimulatory sequences may also be used as comparative examples :

OLIGO 3: TCC ATG <u>ACG T</u>TC CTG <u>ACG T</u>T

OLIGO 4: TCT CCC A<u>GC GTG CGC</u> CAT

The CpG and flanking sequences have been underlined, and there is conserved ACGT, ACG and GCG motifs. The sequences derived from the choline binding regions of pneumococcal proteins have two CpG motifs that repeated 10 or 15 nucleotide bases apart, and a motif based on this nucleotide base distance between two CpGs occurs three times and five times respectively in the lytA and CbpA proteins. However, the published sequences have two CpG motifs that are seven or two nucleotide bases apart.

In one embodiment, when combined with commercially available 23 valent polysaccharide vaccine (Pneumovax®, Pasteur Merieux), CpG adjuvantation significantly augmented the immune response ( IgG antibody) especially to polysaccharide types 19F and 14 when administered Intramuscularly.

Thus advantageously in an embodiment of the present invention it is possible to enhance the efficacy of a commercially available pneumococcal vaccine. This is particularly important in high risk populations, especially those which have sub-optimal antibody responses to the polysaccharides. Such populations may include, but are not limited to, the elderly, patients with any of the following: splenectomy, congenital asplenia, hyposplenia, sickle cell disease, cyclic neutropenia, drug-induced neutropenia, aplastic anaemia, congenital agammaglobulinemia, hypogammaglobulinemia, selective IgG subclass deficiency, multiple myeloma, chronic lymphocytic leukaemia, lymphoma, HIV infection, multifactorial conditions such as glucocorticoid treatment, malnutrition, cirrhosis of the liver, renal insufficiency, diabetes mellitus, alcoholism, chronic disease, hospitalisation, fatigue, stress, cold exposure, prior respiratory infection, influenza; asthma. It may also include healthy adults such as health workers, military trainees, prisoners, or others including school attendees or travellers wishing to ensure full vaccine coverage.

In a preferred application, the CpG adjuvant is used to augment the response to the polysaccharide vaccine when used as booster in children between 6 and 24 months of age that have received their primary immunisation with a multivalent pneumococcal polysaccharide-protein conjugate. Such vaccines utilised for primary immunisation may also advantageously, be adjuvanted with a CpG oligonucleotide. Accordingly in one embodiment there is provided a method of immunisation of a patient comprising administering an effective amount of a vaccine according to the invention.

In a second embodiment there is provided a method of boosting an immune response to a subject previously primed to an antigen by administering a T-independent pneumococcal antigen with a CpG immunostimulatory oligonucleotide.

Other T-independent antigen based vaccines include, but are not limited to, the Vi polysaccharide vaccine against *Salmonella typhi,* the tetravalent meningococcal polysaccharide vaccine (comprising types A, C, W135 and Y), the polysaccharide and modified polysaccharides of group B meningococcus, polysaccharides from *Staphylococcus aureus,* polysaccharides from *Streptococcus agalactae,* polysaccharides from Mycobacteria, eg *Mycobacterium tuberculosis,* such as mannophosphoinisitides trehaloses, mycolic acid, mannose capped arabinomannans, the capsule therefrom and arabinogalactans, polysaccharide from *Cryptococcus neoformans,* the lipopolysaccharides of non-typeable *Haemophilus influenzae,* the lipopolysaccharides of *Moraxella catharralis,* the lipopolysaccharides of *Shigella sonnei,* the lipopeptidophosphoglycan (LPPG) of *Trypanosoma cruzi,* the cancer associated gangliosides GD3, GD2, the tumor associated mucins, especially the T-F antigen, and the sialyl T-F antigen, and the HIV associated polysaccharide that is structurally related to the T-F antigen. Other T independent antigens may be derived from: Salmonella, Vibrio cholera, Escherichia, Chlamydia and T-independent antigens from Plasmodium.

Vaccine preparation is generally described in Pharmaceutical Biotechnology, Vol.6 Vaccine Design - the subunit and adjuvant approach, edited by Powell and Newman, Plenum Press, 1995. Encapsulation within liposomes is described, for example, by Fullerton, US Patent 4,235,877. Conjugation of proteins to macromolecules is disclosed, for example, by Likhite, US Patent 4,372,945 and by Armor et al, US Patent 4,474,757.

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 0.1-1000 μg of polysaccharide or polysaccharide - protein conjugate, preferably 2-100 μg, most preferably 4-40 μg. An optimal amount for a particular vaccine can be ascertained by standard studies involving observation of appropriate immune responses in subjects. Following an initial vaccination, subjects may receive one or several booster immunisation adequately spaced.

The oligonucleotides utilised in the present invention are typically deoxynucleotides. In a preferred embodiment the internucleotide bond in the oligonucleotide is phosphorodithioate, or more preferably phosphorothioate bond, although phosphodiesters are within the scope of the present invention. Other intemucleotide bonds which stabilise the oligonucleotide may be used.

The CpG oligonucleotides utilised in the present invention may be synthesized by any method known in the art (eg EP 468 520) conveniently such oligonucleotides can be synthesized utilising an automated synthesizer. Methods for producing phosphorothioate oligonucleotides or phosphorodithioate are described in US 5,663,153, US 5,278,302 and WO 95/26204.

**Example 1 - CpG adjuvantation of 23-valent Pneumococcal Polysaccharide in mice**

Protection against pneumococcal infection is mediated by IgG antibody to the capsular polysaccharide, along with the deposition of complement that renders the bacteria susceptible to killing by neutrophils via opsonophagocytosis. Thus the protective efficacy of the vaccine may be estimated solely on the basis of IgG antibody induction. Groups of 10 mice were immunised once with a commercial 23-Valent pneumococcal polysaccharide vaccine at 1/10, 1/50 or 1/250 human dose (57.7,11.5 and 2.3 μg total polysaccharide respectively), and adjuvanted with CpG (50 μg of oligo 1), CpG + Alum. Following immunization, serum IgG concentrations to the 4 most important serotype polysaccharides (6B, 14, 19F and 23F) were measured by ELISA every 7 days for 4 weeks.

**Materials and Methods**

The following groups were immunised. (10 balb/c mice per group):

23 Valent at 2.3, 11.5 and 57.5 μg/dose (1/250, 1/50 and 1/10 human dose)
23 Valent + CpG (50 μg) in the same dose range
23 Valent + CpG + Al(OH)3 in the same dose range

**Components used**

| Component | Batch | Concentration μg/ml | Buffer |
|---|---|---|---|
| 23 Valent from Pasteur Mérieux (Pneumovax® 23) | 95K03-HC56630 | 1150 | Saline |
| CpG | Oligo 3 | 5000 | $H_2O$ |
| Al(OH)$_3$ | 96A0089 | 10380 | $H_2O$ |

**Formulation process**

The Pneumovax was diluted in $H_2O$ and 10-fold concentrated 10mM $PO_4$, 150 mM NaCl pH 6.8 to obtain 2.3, 11.5 or 57.7 μg of antigen per dose. CpG was added for 30min and for groups containing Al(OH)$_3$ the formulations were adsorbed for 30 min on either Al(OH)$_3$ (50μg). Thiomersal (50 μg/ml) was added as preservative.

**ELISA**

There were 10 animals per group, but since bleeds were performed every week, only 5 animals per week were bled. ELISA and opsonophagocytosis were performed on pooled sera.

The ELISA was performed to measure murine IgG using the protocol from the WHO Workshop on the ELISA procedure for the quantitation of IgG antibody against *Streptococcus pneumoniae* capsular polysaccharides in human serum. In essence, purified capsular polysaccharide is coated directly on the microtitre plate. Serum samples are pre-incubated with the cell-wall polysaccharide common to all pneumococcus and which is present in ca. 0.5% in pneumococcal polysaccharides purified according to disclosure (EP72513 B1). Jackson ImmunoLaboratories Inc. reagents were employed to detect bound murine IgG. The titration curves were referenced to internal standards (monoclonal antibodies) modeled by logistic log equation. The calculations were performed using SoftMax Pro® software. The maximum <u>absolute</u> error on these results expected to be within a factor of 2. The relative error is less than 30%.

**Results**

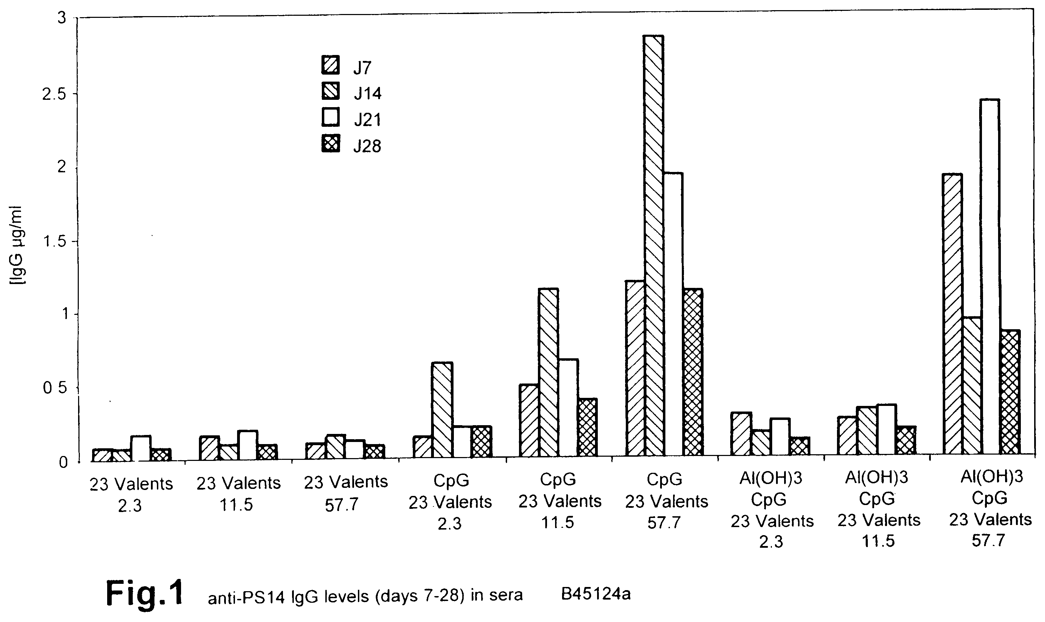

IgG isotype antibodies were found against serotypes 14 and 19G, but not against 6B and 23F, and the results for serotype 14 are presented in figure 1. The response was dose dependent with 1/10 human dose giving the highest response, indicating that the IgG response was specific for the polysaccharide. This is unusual since mice normally only produce IgM against pneumococcal polysaccharides. The peak response was on day 14 post immunisation, which is not unusual since T-independent antigens do not induce memory.

Additional individual analysis were carried out to determine the variance and the statistical significance (data not shown). The response to 1/10 human dose 23-valent was (statistically) significantly increased when adjuvanted with CpG alone (for Type 19, GMC 0.8 compared to 3.7 µg/ml p=0.07; for Type 14, GMC 0.19 compared to 3.4 µg/ml, p=001). This was also true for 1/50 and 1/250 doses when measured against type 14. In addition, responses were significantly increased to type 14 when adjuvanted with CpG+Alum.

The highest response was induced when the vaccine was adjuvanted with CpG alone.

**Example 2 - Effect of CpG adjuvantation on the immunogenicity of tetravalent Pneumococcal PS-PD conjugates in the infant rat model**

The infant rat model was selected since published data showed that the relative immunogenicity of 4 pneumococcal polysaccharide protein conjugates in human infants was more similar to rats than mice. That is 6B<23F<14<19F for infant rats. Infants rats were selected because their immune system may have developmental immaturity similar to that found in human infants.

Infant rats were immunized with Clinical grade lots of Tetravalent pneumococcal polysaccharide-PD[(a)] conjugates in a 5-fold dose range and with the adjuvants CpG and AlPO4+CpG. Oligo 1 was used at a dosage of 100µg. Animals were first immunized when they were 7 days old and received subsequent immunizations 14 and 28 days later. Serology was performed on samples from day 42 (14 days post III) and 56 (28 days post III).

The best adjuvant was CpG alone : it increased geometric mean IgG concentrations and opsonic titers to 6B, 23F and 19F, whereas titers for serotype 14 were comparable to the other adjuvanted preparations. The CpG alone formulation was also able to significantly increase the seroconversion rates to the 6B-PD serotype.

**Materials and Methods**

**Vaccine groups**

The vaccine lot DSP0401x contains Tetravalent PS-PD Clinical-grade lots D6BPJ208 + D14PDJ202 + D19PJ206 + D23PDJ212. ESPL001 contains Tetravalent PS-LPD lots E6BL040P + E14L66P + E19FL033P + E23FL21P.

| Group | Vaccine Lot | Adjuvant | Dose (µg each PS) |
|---|---|---|---|
| | | | |
| 1 | none | CpG | - |
| 2 | DSP0401x | None | 0.1 |
| 3 | DSP0401x | None | 0.5 |
| 4 | DSP0401x | AlPO4 | 0.1 |
| 5 | DSP0401x | AlPO4 | 0.5 |
| 6 | DSP0401x | AlPO4 | 2.5 |
| 7 | ESPL001 | AlPO4 | 0.1 |

(continued)

| Group | Vaccine Lot | Adjuvant | Dose ($\mu$g each PS) |
|---|---|---|---|
| 8 | ESPL001 | AIPO4 | 0.5 |
| 9 | ESPL001 | AIPO4 | 1.25 |
| 10 | DSP0401x | CpG | 0.1 |
| 11 | DSP0401x | CpG | 0.5 |
| 12 | DSP0401x | CpG/AIPO4 | 0.1 |
| 13 | DSP0401x | CpG/AIPO4 | 0.5 |

## Components used

| Component | Batch | Concentration $\mu$g/ml | Buffer |
|---|---|---|---|
| Conjugate PD6B | D6BPDJ208 | 206 | NaCl 0.2M pH 6.5 |
| Conjugate PD14 | D14PDJ202 | 186 | NaCl 0.2M pH 6.5 |
| Conjugate PD19 | D19PDJ206 | 175 | NaCl 0.2M pH 6.5 |
| Conjugate PD23 | D23PDJ212 | 158 | NaCl 0.2M pH 6.5 |
| monovalent PD6B | D6BPDD208 | 100 | NaCl 150mM pH 6.1 |
| monovalent PD14 | D14PDD202 | 100 | NaCl 150mM pH 6.1 |
| monovalent PD19 | D19PDD206 | 100 | NaCl 150mM pH 6.1 |
| monovalent PD23 | D23PDD212 | 96 | NaCl 150mM pH 6.1 |
| monovalent LPD6B | E6BL040P | 50 | NaCl 150mM pH 6.1 |
| monovalent LPD14 | E14FL66P | 50 | NaCl 150mM pH 6.1 |
| monovalent LPD19 | E19FL033P | 50 | NaCl 150mM pH 6.1 |
| monovalent LPD23 | E23FL21P | 50 | NaCl 150mM pH 6.1 |
| Tetravalent LPD | ESPL001 | 5/valence | NaCl 150mM pH 6.1 |
| CpG | Oligo 1, WD1001 | 5000 | $H_2O$ |
| AIPO4 | 97D0045 | 5040 | NaCl 150mMpH 6.1 |

## Formulation Process

### Non adsorbed tetravalents.

The four conjugates are diluted in H2O and 10-fold concentrated NaCl 150mM. Phenoxyethanol (500 $\mu$g/ml) is added as preservative.

If CpG is needed, the oligonucleotide is added to the non adsorbed tetravalent. The isotonicity and the dilution when needed are ensured by NaCl.

### Adsorbed tetravalents

The four concentrated, adsorbed monovalents are diluted in H2O and 10-fold concentrated 150mM NaCl before addition of AIPO4. Phenoxyethanol (500 $\mu$g/ml) is added as preservative.

If dilutions are needed, the tetravalents are diluted in AIPO4 at 1 mg/ml. These diluents are prepared in NaCl 150 mM.

If CpG is needed, the oligonucleotide is added to the adsorbed tetravalent. The isotonicity is ensured by addition of NaCl 1500 mM and if dilutions are required, diluents of AlPO4 at 1.3 or 1.8 mg/ml in NaCl are added.

All the formulations are prepared in non siliconized glass vials.

**Immunisation Protocol**

Infant rats were randomised to different mothers and were 7 days old when they received the first immunisation. They received subsequent immunisations 14 and 28 days later. Bleeds were performed on day 42 (14 days post III) and 56 (28 days post III). All vaccines were injected s.c., and there were 10 rats per vaccine group.

**ELISA**

The ELISA was performed to measure rat IgG using the protocol derived from the WHO Workshop on the "ELISA procedure for the quantitation of IgG antibody against Streptococcus pneumoniae capsular polysaccharides in human serum". In essence, purified capsular polysaccharide is coated directly on the microtitre plate. Serum samples are pre-incubated with the cell-wall polysaccharide common to all pneumococcus and which is present in ca. 0.5% in pneumococcal polysaccharides purified. Jackson ImmunoLaboratories Inc. reagents were employed to detect bound rat IgG. The titration curves were referenced to the titration curve of a reference serum modeled by logistic log equation. The calculations were performed using SoftMax Pro (trademark) software. The standard sera were calibrated using a method of corollary response, and the values were demonstrated to correspond to estimations of Ig concentrations found by immunoprecipitation (Ref. 21).

**Opsonophagocytosis**

The opsonophagocytic assay was performed following the CDC protocol (Streptococcus pneumoniae Opsonophagocytosis using Differentiated HL60 cells, version 1.1). Modification included the use of in-house pneumococcal strains, and the phagocytic HL60 cells were replaced by purified human PMN. Rat polyclonal sera were included as a positive control.

**Results**

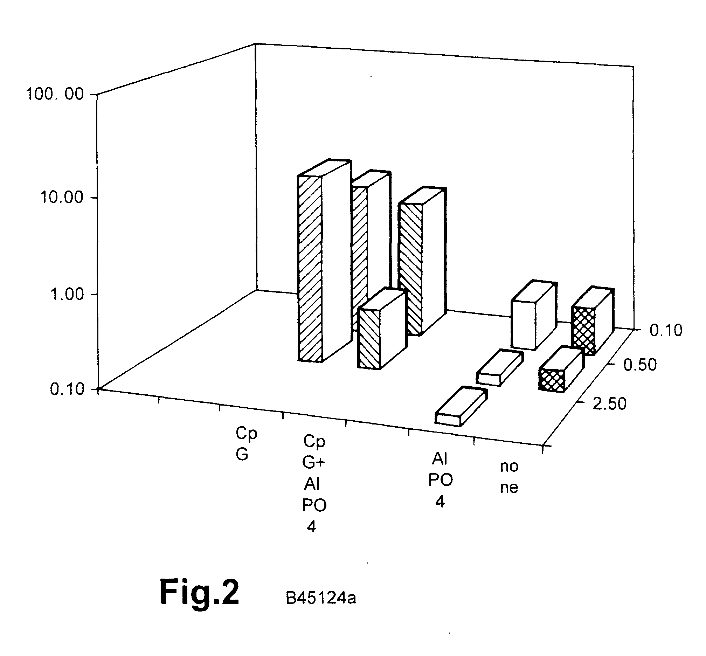

Figure 2 shows the geometric mean IgG concentrations elicited against serotype 6B by the tetravalent combinations described in the materials and methods. For clarity, the axes are divided by adjuvant and dose. Similar results were obtained against the serotypes 19F and 23F, but type 14 had a more uniform reponse to all adjuvants and doses.

The biological activity of the pooled antisera from each adjuvant group and dose was measured by opsonophagocytosis. The opsonic activity relative to the concentration of IgG will give an estimate of the functional activity of the antisera. The values, shown in Table 1 show that all adjuvants induce antibody that has approximately the same capacity to opsonise pnemococci. Thus CpG aids in the induction of specific antibody, and increases in antibody concentration correlate with increases in protective efficacy.

**Conclusion**

- AlPO4 (compared to no adjuvant) significantly increases the seroconversion rate, geometric mean IgG concentration, opsonic activity and immunological memory to tetravalent PS-PD.

- The 0.1 µg dose is significantly more immunogenic than 0.5 µg dose for serotypes 6B, 19F and 23F PS-PD conjugates on AlPO4.

- IgG concentrations are significantly increased against serotypes 6B, 19F and 23F when the conjugate vaccine is adjuvanted with CpG compared to AlPO4. This is confirmed by increased seroconversion rates and increased opsonophagocytic titres.

Table 1.

| Relative opsonic activity (Concentration of IgG required for 50% killing of pneumococcus) compared by serotype and adjuvant. | | | | | | | |
|---|---|---|---|---|---|---|---|
| **Vaccine** | **Adjuvant** | **Dose** | **Concentration of IgG required for 50% killing** | | | | **Average by adjuvant** |
| | | µg | 6B | 14 | 19F | 23F | |
| DSP0401x | none | 0,1 | 0,32 | 0,30 | 0,30 | 0,37 | 0.26±0.14 |
| | | 0,5 | No Value | 0,015 | No Value | No Value | |
| DSP0401x | AIPO4 | 0,1 | 0,02 | 0,31 | 0,40 | 0,09 | 0.20±0.15 |
| | | 0,5 | No Value | 0,05 | 0,22 | No Value | |
| | | 2,5 | No Value | 0,32 | #VAL | No Value | |
| ESPL001 | AIPO4 | 0,1 | 0,08 | 0,46 | No Value | 0,22 | 0.35 ±0.27 |
| | | 0,5 | 0.11 | 0,71 | 0,75 | 0,08 | |
| | | 1,25 | 0,10 | 0,55 | 0,66 | 0,20 | |
| DSP0401x | CPG | 0,1 | 0,42 | 0,15 | No Value | 0,20 | 0.24±0.10 |
| | | 0,5 | 0,21 | 0,30 | No Value | 0,17 | |
| DSP0401x | CPG / AIPO4 | 0,1 | 0,27 | 0,10 | No Value | 0,21 | 0.20±0.14 |
| | | 0,5 | No Value | 0,10 | 0,44 | 0,09 | |
| Average | by serotype | | 0.19 ± 0.14 | 0.29 ± 0.20 | 0.45 ± 0.20 | 0.18 ± 0.09 | |

## Example 3 - Effect of CpG adjuvantation on the immunogenicity of 11-Valent Pneumococcal PS-PD conjugates in the infant rat model

Example 2 showed that CpG adjuvantation of conjugate vaccines resulted in fold increases of the order of 5 to 10 times higher than with conventional adjuvants (Aluminium). In order to determine whether these effects were dependent on the Oligo sequence, dosage, or formulation, further experimentation was undertaken.

CpG OLIGO 2 was selected and used at a lower dosage, that is 1 and 10 µg. It was also adsorbed onto Al (OH)3, and combined with the conjugate vaccines.

In addition, since the immunological characteristics of each polysaccharide may be different, 11 serotypes were tested.

## Material and Methods

Table 2.

| Choice of pneumococcal PS-PD lots | | | | | | | | | | | |
|---|---|---|---|---|---|---|---|---|---|---|---|
| Serotype | 1 | 3 | 4 | 5 | 6B | 7F | 9V | 14 | 18C | 19F | 23F |
| Lot number | 017 | 040 | 218 | 024 | 209 | 019 | 222 | 204 | 221 | 207 | 213 |

## Formulation

To examine the effect of different advanced adjuvants, the dosage of conjugate was held constant at 0.1 µg of each polysaccharide, and the adjuvants AlPO4, Al(OH)3and CpG were formulated in different dosages and combinations. In total, 10 different combinations were tested, including no adjuvant at all. These are listed numerically in Table 3 for reference.

**Preparation of diluents**

Two diluents were prepared in NaCl 150 mM/phenoxy

A: AlPO$_4$ at 1mg/ml.
B: CpG on Al(OH)$_3$ at 200 and 1000 μg/ml respectively.Weight ratio
CpG/Al(OH)$_3$ = 1/5

**Preparation of adsorbed undecavalent**

The eleven concentrated, adsorbed PS-PD monovalents were mixed at the right ratio. The complement of AlPO4 was added. When needed, CpG (CpG adsorbed on Al(OH)3) or diluent was added.

**Preparation of non-adsorbed undecavalent**

The eleven PS-PD conjugates were mixed and diluted at the right ratio in NaCl 150 mM pH 6.1, phenoxy. When needed, CpG was added either as a solution (non adsorbed) or as CpG adsorbed on Al(OH)3.
The formulations for all injections were prepared 18 days before the first administration.

Table 3.

| Summary Table of Adjuvant Formulations tested with 11-Valent Pneumococcal PS-PD in Infant Rats | | | | |
|---|---|---|---|---|
| Group | AlPO4 | CpG | Al(OH)3 | Description |
| 1 | | | | None |
| 2 | 100 | | | AlPO4 |
| 3 | | 1 | | CpG low |
| 4 | | 10 | | CpG High |
| 5 | | 1 | 4.5 | CpG ads low |
| 6 | | 10 | 50 | CpG ads high |
| 7 | 100 | 1 | | CpG low Coni ads |
| 8 | 100 | 10 | | CpG Hi Coni ads |
| 9 | 95 | 1 | 4.5 | CpG&Conj ads low |
| 10 | 50 | 10 | 50 | CpG&Conj ads Hi |

**Immunisation Protocol**

Infant OFA rats were randomised to different mothers and were 7 days old when they received the first immunisation. They received 2 additional immunisations 14 and 28 days later. A bleed as performed on day 56 (28 days post III). All vaccines were injected s.c., and there were 10 rats per vaccine group.

**ELISA**

The ELISA was performed to as described in example 2.

**Opsonophagocytosis**

The opsonophagocytic assay was performed following the CDC protocol (Streptococcus pneumoniae Opsonophagocytosis using Differentiated HL60 cells, version 1.1). Modification included the use of in-house pneumococcal strains, and the phagocytic HL60 cells were replaced by purified human PMN. In addition, 3 mm glass beads were added to the microtitre wells to increase mixing, and this allowed reduction of the phagocyte:bacteria ratio which was recommended to be 400.

**Results**

Tables 4 to 7 below show the geometric mean IgG concentration, seroconversion rate and arithmetic mean opsonophagocytic titre determined for 4 serotypes of pneumococci after immunisation with an 11Valent pneumococcal PS-Protein D conjugate vaccine adjuvanted with different formulations of CpG OLIGO 2. Compared to no adjuvant, 10 µg CpG induced significant higher IgG concentrations for all serotypes. CpG induced significantly higher IgG concentrations than AlPO4 for serotypes 1, 6B, 18C and 19F.

For comparison, included in the Tables are the results from Example2 using OLIGO 1. There are no significant differences in the IgG responses induced by the two OLIGO sequences when OLIGO 2 is used at 10 µg. However, OLIGO 2 at 1 µg shows no immunostimulatory effects evidenced in that the induced IgG concentrations are not significantly different from without CpG.

Adsorption of OLIGO 2 on Al(OH)3 reduces the immunostimulatory effect, and the induction of antibody is not significantly different than AlPO4 as adjuvant.

TABLE 4

| Serotype 6B Geometric Mean IgG Concentration, Seroconversion, and Mean Opsonic Titre on Day 28 Post III Immunisation of Infant Rats with 11-Valent PS-PD using Different Adjuvants (And Comparison with Tetravalent Immunisation, Example 2) | | | | | | | | | |
|---|---|---|---|---|---|---|---|---|---|
| Group | Al PO 4 µg | Oligo 1 µg | Oligo 2 µg | 6B GMC IgG µg/ml | 6B Seroconversion | 6B Opso Titre* | 6B GMC IgG µg/ml | 6B Seroconversion | 6B Opso Titre* |
| | | | | Example 2 | | | Example 3 | | |
| 1 | | | | 0.047 | 2/10 | 12.5 | 0.004 | 1/10 | < 6.25 |
| 2 | 100 | | | 0.048 | 4/10 | 65 | 0.019 | 4/10 | <6.25 |
| 3 | | | 1 | | | | 0.003 | 1/10 | < 6.25 |
| 4 | | | 10 | | | | 1.682 | 10/10 | 157 |
| | | 100 | | 0.63 | 8/10 | 48 | | | |
| 5 | | | 1 µg on Al(OH)3 | | | | 0.015 | 6/10 | < 6.25 |
| 6 | | | 10 µg on Al(OH)3 | | | | 0.007 | 3/10 | < 6.25 |
| 7 | 100 | | 1 | | | | 0.029 | 7/10 | <6.25 |
| 8 | 100 | | 10 | | | | 0.469 | 9/10 | 77 |
| | 100 | 100 | | 0.46 | 7/10 | 75 | | | |
| 9 | 95 | | 1 µg on Al(OH)3 | | | | 0.040 | 5/10 | 38 |
| 10 | 50 | | 10 µg on Al(OH)3 | | | | 0.022 | 7/10 | <6.25 |

TABLE 5

| Serotype 14 Geometric Mean IgG Concentration, Seroconversion, and Mean Opsonic Titre on Day 28 Post III Immunisation of Infant Rats with 11-Valent PS-PD using Different Adjuvants (And Comparison with Tetravalent Immunisation, Example 2) | | | | | | | | | |
|---|---|---|---|---|---|---|---|---|---|
| Group | Al PO4 4 µg | Oligo 1 µg | Oligo 2 µg | 14 GMC IgG µg/ml | 14 Seroconversion | 14 Opso Titre* | 14 GMC IgG µg/ml | 14 Seroconversion | 14 Opso Titre* |
| | | | | Example 2 | | | Example 3 | | |
| 1 | | | | 0.046 | 3/10 | 64 | 0.022 | 3/10 | < 6.25 |
| 2 | 100 | | | 0.99 | 10/10 | 88 | 0.237 | 8/10 | 27 |
| 3 | | | 1 | | | | 0.035 | 4/10 | <6.25 |
| 4 | | | 10 | | | | 0.361 | 10/10 | 88 |
| | | 100 | | 0.66 | 9/10 | 295 | | | |
| 5 | | | 1 µg on Al(OH)3 | | | | 0.093 | 9/10 | < 6.25 |
| 6 | | | 10 µg on Al (OH)3 | | | | 0.155 | 9/10 | 27 |
| 7 | 100 | | 1 | | | | 0.134 | 7/10 | < 6.25 |
| 8 | 100 | | 10 | | | | 2.028 | 10/10 | 188 |
| | 100 | 100 | | 2.3 | 10/10 | 888 | | | |
| 9 | 95 | | 1 µg on Al(OH)3 | | | | 0.140 | 6/10 | 138 |
| 10 | 50 | | 10 µg on Al (OH)3 | | | | 0.196 | 10/10 | < 6.25 |

TABLE 6

| Serotype 19F Geometric Mean IgG Concentration, Seroconversion, and Mean Opsonic Titre on Day 28 Post III Immunisation of Infant Rats with 11-Valent PS-PD using Different Adjuvants (And Comparison with Tetravalent Immunisation, Example 2) | | | | | | | | | |
|---|---|---|---|---|---|---|---|---|---|
| Group | Al PO 4 µg | Oligo 1 µg | Oligo 2 µg | 19F GMC IgG µg/ml | 19F Seroconversion | 19F Opso Titre* | 19F GMC IgG µg/ml | 19F Seroconversion | 19F Opso Titre* |
| | | | | Example 2 | | | Example 3 | | |
| 1 | | | | 0.04 | 2/10 | 64 | 0.021 | 2/10 | <6.25 |
| 2 | 100 | | | 1.07 | 9/10 | 367 | 0.222 | 7/10 | 79 |
| 3 | | | 1 | | | | 0.015 | 3/10 | <6.25 |
| 4 | | | 10 | | | | 4.287 | 10/10 | 415 |
| | | 100 | | 12. | 10/10 | > 1600 | | | |

TABLE 6   (continued)

| Serotype 19F Geometric Mean IgG Concentration, Seroconversion, and Mean Opsonic Titre on Day 28 Post III Immunisation of Infant Rats with 11-Valent PS-PD using Different Adjuvants (And Comparison with Tetravalent Immunisation, Example 2) | | | | | | | | | |
|---|---|---|---|---|---|---|---|---|---|
| Group | Al PO 4 µg | Oligo 1 µg | Oligo 2 µg | 19F GMC IgG µg/ml | 19F Seroconversion | 19F Opso Titre* | 19F GMC IgG µg/ml | 19F Seroconversion | 19F Opso Titre* |
| | | | | Example 2 | | | Example 3 | | |
| 5 | | | 1 µg on Al (OH)3 | | | | 0.417 | 9/10 | 32 |
| 6 | | | 10 µg on Al (OH)3 | | | | 1.612 | 9/10 | 94 |
| 7 | 100 | | 1 | | | | 0.441 | 10/10 | 135 |
| 8 | 100 | | 10 | | | | 9.475 | 10/10 | > 1600 |
| | 100 | 100 | | 11.0 | 10/10 | >1600 | | | |
| 9 | 95 | | 1 µg on Al (OH)3 | | | | 0.438 | 9/10 | 377 |
| 10 | 50 | | 10 µg on Al (OH)3 | | | | 0.258 | 7/10 | 165 |

TABLE 7

| Serotype 23F Geometric Mean IgG Concentration, Seroconversion, and Mean Opsonic Titre on Day 28 Post III Immunisation of Infant Rats with 11-Valent PS-PD using Different Adjuvants (And Comparison with Tetravalent Immunisation, Example 2) | | | | | | | | | |
|---|---|---|---|---|---|---|---|---|---|
| Group | Al PO 4 µg | Oligo 1 µg | Oligo 2 µg | 23F GMC IgG µg/ml | 23F Seroconversion | 23F Opso Titre* | 23F GMC IgG µg/ml | 23F Seroconversion | 23F Opso Titre* |
| | | | | Example 2 | | | Example 3 | | |
| 1 | | | | 0.06 | 2/10 | <6.25 | 0.152 | 3/10 | <6.25 |
| 2 | 100 | | | 0.29 | 10/10 | 70 | 0.56 | 8/10 | <6.25 |
| 3 | | | 1 | | | | 0.114 | 4/10 | <6.25 |
| 4 | | | 10 | | | | 1.305 | 9/10 | 192 |
| | | 100 | | 2.0 | 10/10 | 454 | | | |
| 5 | | | 1 µg on Al (OH)3 | | | | 0.28 | 7/10 | <6.25 |
| 6 | | | 10 µg on Al (OH)3 | | | | 0.107 | 2/10 | <6.25 |
| 7 | 100 | | 1 | | | | 0.243 | 4/10 | < 6.25 |

TABLE 7   (continued)

| Serotype 23F Geometric Mean IgG Concentration, Seroconversion, and Mean Opsonic Titre on Day 28 Post III Immunisation of Infant Rats with 11-Valent PS-PD using Different Adjuvants (And Comparison with Tetravalent Immunisation, Example 2) | | | | | | | | | |
|---|---|---|---|---|---|---|---|---|---|
| Group | Al PO 4 µg | Oligo 1 µg | Oligo 2 µg | 23F GMC IgG µg/ml | 23F Seroconversion | 23F Opso Titre* | 23F GMC IgG µg/ml | 23F Seroconversion | 23F Opso Titre* |
| | | | | Example 2 | | | Example 3 | | |
| 8 | 100 | | 10 | | | | 1.545 | 9/10 | 862 |
| | 100 | 100 | | 1.1 | 10/10 | 265 | | | |
| 9 | 95 | | 1 µg on Al (OH)3 | | | | 0.255 | 3/10 | 44 |
| 10 | 50 | | 10 µg on Al (OH)3 | | | | 0.331 | 6/10 | <6.25 |

**Example 5 Influence of CpG on Boosting with Polysaccharide after Priming with Polysaccharide-Conjugate Vaccines, and on Priming with Polysaccharide.**

The previous examples have demonstrated the ability of CpG to adjuvant the immune response to T-independent antigens, and to T-independent antigens coupled to a protein carrier. There remained to be considered whether CpG could adjuvant a memory response elicited by boosting with a T-independent antigen after priming with T-dependent antigen. It was of further interest to determine if CpG could act to induce priming by a T-independent antigen.

To determine these effects, mice were primed with either pneumococcal polysaccharide, or CpG adjuvanted pneumococcccal polysaccharide, or Protein D conjugate pneumococcal polysaccharide.

**Immunisation Protocol**

Six to 8 week old balb/c mice were immunise subcutaneously with the vaccine formulations described below. The dosage was 1 µg per polysaccharide for both conjugated and non-conjugated formulations. A test bleed was performed 14 days later to measure IgG concentrations. After 56 days, another test bleed was performed, and then a booster vaccination was given, and a final test bleed was performed 14 days later, that is 70 days after the first immunisation.

| Group | Prime | Boost |
|---|---|---|
| 1 | Saline | Conjugate |
| 2 | PS | PS |
| 3 | PS/Cp | PS |
| 4 | PS | Conjugate |
| 5 | PS/Cp | Conjugate |
| 6 | Conjug | PS |
| 7 | Conjug | PS/CpG |
| 8 | Conjug | Conjugate |

**Components used**

| Component | Batch | Conc µg/ml | Buffer | Adsorption | PS conc after adsorption µg/ml | Buffer after adsorption |
|---|---|---|---|---|---|---|
| PS6b | 6b/24 | 2000 | NaCl 150mM | | | |
| PS14 | 14/19 | 2000 | NaCl 150mM | | | |
| PS19 | 19f/26b | 2000 | NaCl 150mM | | | |
| PS23 | 23f/29 | 2000 | NaCl 150mM | | | |
| Conjugate PDPS6B | D6BPDJ 209 | | | MBSP9801 | 100 | NaCl 150mM pH 6.1/phenoxy |
| Conjugate PDPS14 | D14PDJ 204 | | | MBSP9801 | 100 | NaCl 150mM pH 6.1/phenoxy |
| Conjugate PDPS19 | D19PDJ 207 | | | MBSP9801 | 100 | NaCl 150mM pH 6.1/phenoxy |
| Conjugate PDPS23 | D23PDJ 213 | | | MBSP9801 | 100 | NaCl 150mM pH 6.1/phenoxy |
| CpG | Oligo 1 | 5000 | H2O | | | |
| St Pn AIPO4 diluent | 97D004 5 | | | | 1000 | NaCl 150mM pH 6.1/phenoxy |

**Formulation process**

Preparation of 4 concentrated, adsorbed monovalents (PS-PD conjugates)

The concentrated, adsorbed monovalents were prepared according the procedure described above in Example 2.

**Preparation of tetravalent (PS-PD conjugates)**

The four concentrated, adsorbed monovalents were mixed at the right ratio (1µg of each valence/dose) and diluted in NaCl pH6.1. The complement of AIPO4 (10µg/dose) was added as a diluent at 1mg/ml in NaCl 150mM pH6.1 containing 5mg/ml of phenoxyethanol.

Preparation of non-conjugated, non-adsorbed, tetravalent with or without CpG (free PS)

The four free PS were mixed at the right ratio (1µg of each valence/dose) and diluted in NaCl pH6.1. When needed, CpG (100µg/dose) was added. Five mg/ml of phenoxyethanol were added as preservative.
The formulations for both injections were prepared 6 days before the first administration in non siliconized glass vials.

**Formulation process**

Preparation of 4 concentrated, adsorbed monovalents (PS-PD conjugates)

The concentrated, adsorbed monovalents were prepared according the procedure describe above.

**Preparation of tetravalent (PS-PD conjugates)**

The four concentrated, adsorbed monovalents were mixed at the right ratio (1µg of each valence/dose). The complement of AlPO4 (10µg/dose) was added as a diluent at 1mg/ml in NaCl 150mM pH6.1 containing 5mg/ml of phenoxyethanol.

**Preparation of non conjugated, non adsorbed, tetravalent with or without CpG (free PS)**

The four free PS were mixed at the right ratio (1µg of each valence/dose) and diluted in NaCl pH6.1. When needed, CpG was added. Five mg/ml of phenoxyethanol were added as preservative.

The formulations for both injections were prepared 6 days before the first administration in non siliconized glass vials.

**ELISA**

The ELISA was performed as described in Example 1

**Results**

The results of this experiment are the priming and the boosting. The results of the priming were consistent with previous observations (Example 1) in that increased seroconversion and higher IgG concentrations were found in mice that were immunised with CpG adjuvanted polysaccharide compared to plain polysaccharide. As was found in Example 1, the increases in type 14 IgG concentration with CpG adjuvantation are statistically significant compared to PS alone, and the increases for type 19F approach significance. However, the IgG concentrations with CpG adjuvantation were not as high as observed in Example 1. To explain this difference, only two differences in the experiments were made, the valency of the vaccine (23 valent versus 4 valent) and the route of immunisation (intramuscular versus subcutaneous). Since the reduction of valence is not expected to decrease immunogenicity, the evidence indicates that the route of immunisation is important for optimal CpG adjuvantation of T-independent antigens. This is consistent with a recent publication which disclosed a failed attempt to use CpG adjuvantation of a plain polysaccharide vaccine. The route of immunisation employed was interperitoneal (Threadgill et al Vaccine 1998 Vol 16(1) p76).

|  | Seroconversion | GMC |
|---|---|---|
| PS 14 | 2/20* | 0.07 |
| PS14/CpG | 12/20*δ | 0.15 |
| Conjugate | 24/30δ | 1.04 |
| PS19F | 1/20ξ | 0.08 |
| PS19F CpG | 4/20ξω̄ | 0.10 |
| Conjugate | 22/30ω̄ | 0.35 |

* $p = 0.001$ Fisher's exact test
δ $p = 0.11$ Fisher's exact test
ξ $p = 0.17$ Fisher's exact test
ω̄ $p < 0.001$ Fisher's exact test

In the second part of this experiment, animals primed with either PS, PS/CpG or conjugate vaccine, were boosted with PS, or with PS/CpG or with conjugate. To normalise the data for comparison, the fold increase in IgG was determined 14 days after the booster was given, and the number of animal showing an increase in antibody concentration were counted as responders.

| Prime | Boost | Geometric Fold Increase | Positive responders |
|---|---|---|---|
| PS | PS | 1.7* | 5/10 |
| PS/CpG | PS | 2.8* | 6/10 |

* $p = 0.09$ Student's t-test

(continued)

| Prime | Boost | Geometric Fold Increase | Positive responders |
|---|---|---|---|
| Conjugate | PS | 0.78ξ | 1/10 δ |
| Conjugate | PS/CpG | 1.7ξ | 6/10 δ |
| Conjugate | Conjugate | 4.2 | 7/10 |

ξ p = 0.12 Student's t-test

δ p = 0.03 Fisher's exact test

**Discussion**

This example confirms the results presented in Example 1, but has revealed that the mode of immunisation may be important for optimal immunity. In an extension of the experiment to boosting and memory, two interesting characteristics of CpG adjuvantation are demonstrated. The first is that priming with PS adjuvanted with CpG leads to a higher fold increase upon boosting with polysaccharide, and there is a trend towards statistical significance. This would indicate that CpG was able to induce better memory. The second characteristic is that CpG can adjuvant a memory response induced by polysaccharide in animals primed with conjugate vaccine.

**Conclusions**

CpG is able to induce in mice an antibody isotype switch against non-conjugated polysaccharides. The magnitude of the IgG response is higher with CpG.

**Claims**

1.  A vaccine composition comprising an immunostimulatory CpG oligonucleotide and a polysaccharide antigen from Streptococcus pneumoniae.

2.  A vaccine composition as claimed in claim 1, wherein the polysaccharide is conjugated to a carrier protein.

3.  A vaccine composition as claimed in claim 1 or 2 wherein the immunostimulatory CpG oligonucleotide has at least one intemucleotide bond, selected from phosphodiesters, phosphorodithioate and phosphorothioate.

4.  A vaccine composition as claimed in any of the preceding claims wherein the CpG oligonucleotide is:

TCT CCC AGC GTG CGC CAT

5.  A vaccine composition as claimed in any preceding claim for use in medicine.

6.  A use of the vaccine composition as claimed in any preceding claim in the manufacture of a medicament for inducing an immune response to a polysaccharide antigen from Streptococcus pneumoniae.

**Patentansprüche**

1.  Impfstoffzusammensetzung, die ein immunstimulatorisches CpG-Oligonucleotid und ein Polysaccharid-Antigen aus Streptococcus pneumoniae umfasst.

2.  Impfstoffzusammensetzung gemäß Anspruch 1, worin das Polysaccharid mit einem Trägerprotein konjugiert ist.

3.  Impfstoffzusammensetzung gemäß Anspruch 1 oder 2, worin das immunstimulatorische CpG-Oligonucleotid wenigstens eine Internucleotidbindung hat, die aus Phosphodiestern, Dithiophosphat und Thiophosphat ausgewählt ist.

4. Impfstoffzusammensetzung gemäß einem der vorhergehenden Ansprüche, worin das CpG-Oligonucleotid:

<div align="center">

TCT CCC AGC GTG CGC CAT

</div>

ist.

5. Impfstoffzusammensetzung gemäß jedem vorhergehenden Anspruch zur Verwendung in der Medizin.

6. Verwendung der Impfstoffzusammenesetzung gemäß jedem vorhergehenden Anspruch in der Herstellung eines Medikaments zur Induzierung einer Immunreaktion auf ein Polysaccharid-Antigen aus Streptococcus pneumoniae.

**Revendications**

1. Composition de vaccin comprenant un oligonucléotide CpG immunostimulant et un antigène polysaccharide provenant de Streptococcus pneumoniae.

2. Composition de vaccin selon la revendication 1, dans laquelle le polysaccharide est conjugué à une protéine porteuse.

3. Composition de vaccin selon la revendication 1 ou 2, dans laquelle l'oligonucléotide CpG immunostimulant a au moins une liaison internucléotidique, choisie parmi les phosphodiesters, le phosphorodithioate et le phosphorothioate.

4. Composition de vaccin selon l'une quelconque des revendications précédentes, dans laquelle l'oligonucléotide CpG est :

<div align="center">

TCT CCC AGC GTG CGC CAT

</div>

5. Composition de vaccin selon l'une quelconque des revendications précédentes, à utiliser en médecine.

6. Utilisation de la composition de vaccin selon l'une quelconque des revendications précédentes, dans la fabrication d'un médicament destiné à induire une réponse immunitaire à un antigène polysaccharide provenant de Strepto-coccus pneumoniae.

**Fig.1** anti-PS14 IgG levels (days 7-28) in sera    B45124a

**Fig.2**    B45124a